# EUROPÄISCHE PATENTSCHRIFT

(11) **EP 3 478 655 B1**
(45) Veröffentlichungstag und Bekanntmachung des Hinweises auf die Patenterteilung: **30.09.2020**
(21) Anmeldenummer: 17727604.5
(22) Anmeldetag: 06.06.2017
(51) Int. Cl.: C07C 231/02, C07C 231/12, C07C 233/47, C07C 233/49, C07C 233/36

(54) **VERFAHREN ZUR HERSTELLUNG VON TENSIDEN**
METHOD FOR PRODUCING SURFACTANTS
PROCÉDÉ DE PRODUCTION DE TENSIOACTIFS

(30) Priorität: 29.06.2016 EP 16176790
(43) Veröffentlichungstag der Anmeldung: 08.05.2019
(73) Patentinhaber: Evonik Operations GmbH, 45128 Essen (DE)
(72) Erfinder: LIEBIG, Stefan Julian, 40477 Düsseldorf (DE); SCHUCH, Dominik, 40221 Düsseldorf (DE); VON HOF, Jan Marian, 44789 Bochum (DE); BRANDT, Kathrin Daniela, 40476 Düsseldorf (DE); VOGT, Maximilian, 45143 Essen (DE); WENK, Hans Henning, 45470 Mülheim an der Ruhr (DE)
(74) Vertreter: Evonik Patent Association
(86) Internationale Anmeldenummer: PCT/EP2017/063668
(87) Internationale Veröffentlichungsnummer: WO 2018/001680

(56) Entgegenhaltungen:
- DE-A1- 2 004 099
- US-A1- 2015 335 555

## Beschreibung

### Gebiet der Erfindung

Gegenstand der Erfindung ist ein Verfahren, bei dem eine Aminosäure mit einem Acylgruppendonor unter Erhalt einer N-Acylaminosäure in Gegenwart eines Amins umgesetzt wird.

### Stand der Technik

N-Acylaminosäuren sind besonders milde anionische Tenside und dadurch als interessante Inhaltsstoffe für kosmetische Formulierungen bekannt. Einer breiteren Verwendung stehen jedoch ein aufwendiger Herstellprozess und damit verbundene hohe Kosten entgegen.

Amphotenside, wie insbesondere Betaine, bilden einen wichtigen Baustein kosmetischer Formulierungen und sind bekannt für die Ausbildung eines dichten, weichen Schaums, der eine hohe Stabilität aufweist. Weiterhin werden sie aufgrund ihrer guten Bioabbaubarkeit und geringen Toxizität in großem Maße in der kosmetischen Industrie eingesetzt. Auch amphotere Tenside sind für ihre Mildheit bekannt, und sie können als amphotere Tenside starke Synergien mit anionischen Tensiden wie z.B. SLS oder SLES ausbilden. Aus diesem Grund bilden SLES/Betain-Tensidgemische den Standard im Markt.

Die Synergien zwischen Amphotensiden und anionischen Tensiden können unterschiedlicher Natur sein und beispielsweise Einfluss auf die Verdickung, das Schaumverhalten oder die Mildheit einer Mischung haben.

Auch typische kosmetische Formulierungen die sowohl N-Acylaminosäuren als auch Betaine enthalten sind auf dem Markt präsent. Diese entsprechen dem heutigen Trend sulfatfreie und PEGfreie Formulierungen zu entwickeln.

US8263538 offenbart die Verwendung von N-Acylaminosäuren in Kombination mit amphoteren/zwitterionischen Tensiden um eine sehr milde Formulierung zu erzielen. US20150335555 offenbart die Zusammensetzung einer sulfatfreien kosmetischen Formulierung bestehend aus zwitterionischen/amphoteren Tensiden, N-Acylglycinaten und nichtionischen Tensiden.

US20150141466 offenbart die Verwendung von Aminosäuretensiden und Betainen in Kombination mit N-Methyl-N-Acylglucaminen.

US4772424 offenbart eine Formulierung die eine spezielle Zusammensetzung aus Sulfat/Sulfonat, Betain und Sarkosinat enthält, um eine isotrope Mischung zu erhalten.

N-Acylaminosäuren werden kommerziell zurzeit ausschließlich aus Säurechloriden und Aminosäuren nach der Schotten-Baumann Reaktion gewonnen, wie beispielsweise in US6703517 offenbart. Säurechloride werden durch giftige Chlorierungsmittel hergestellt, es werden Abfallströme erzeugt, was die so gewonnen Säurechloride zu teuren Vorstufen werden lässt. Die verwendeten Chlorierungsmittel, wie beispielsweise Phosphorchloride, werden in energieaufwendigen und umweltbelastenden Prozessen hergestellt.

Bei der Schotten-Baumann Route entstehen stöchiometrische Mengen NaCl, die in der Produktmischung verbleiben.

Oxidationsreaktionen von Amidoalkoholen zu N-Acylaminosäuren wurden durch TEMPO katalysierte Verfahren in der US7439388 sowie durch heterogene Katalyse in der WO2008019887 offenbart. Die Ausbeuten und Selektivitäten dieser Verfahren und die Verwendung von teuren Katalysatoren machen das Verfahren unattraktiv.

Weiterhin wurde die direkte Synthese der N-Acylaminosäuren aus Fettsäuren und Aminosäuren in EP1672055, US3836551, DE4408957 offenbart. Bei diesem Verfahren treten oft Nebenprodukte wie Di-, Tri- oder Tetrapeptide auf, hohe Temperaturen sind erforderlich, das Verhältnis von Fettsäure zu Aminosäure ist in manchen Fällen ungünstig oder die Verwendung von Lösungsmitteln ist notwendig.

Die Darstellung von N-Acylaminosäuren gelingt auch aus Fettsäureestern (Alkyl- oder Polyolestern), wie beispielsweise in US5856538, WO9507881, DE4408957, US4380646, US5898084, JP57/058653 und US20050027120 offenbart. Die beiden Ausgangsverbindungen sind aufgrund ihrer Polaritäten nicht kompatibel, bei erfolgreicher Umsetzung erstarrt das Produkt und es lässt sich nicht ohne Zersetzung schmelzen. Im Falle einer destillativen Nebenkomponenten-Abtrennung wie im Beispiel von Methanol bei Methylestern kann starkes Schäumen auftreten. Nebenprodukte werden gebildet und bei der Verwendung eines Lösungsmittels ist dessen Abtrennung schwierig.

Das N-Acylierungsverfahren kann durchgeführt werden, indem ein Polyol wie beispielsweise Glycerin als Reaktionsmedium eingesetzt wird. Dieses führt dazu, dass das erstarrende Produkt während der Reaktion weiter rührbar bleibt. Das Glycerin verbleibt nach der Reaktion in der Produktmischung, siehe hierzu zum Beispiel WO2013014264, WO2013014265, WO2013014266, WO2013014267 und WO2013014268. In diesem Verfahren wird ein festes Konzentrat hauptsächlich bestehend aus N-Acylaminosäure und Glycerin erhalten. WO2014008103 offenbart eine andere Methode, bei der eine wässrige Lösung aus N-Acylaminosäure erhalten wird.

DE2004099 offenbart ein Verfahren zur Herstellung von Salzen von N-Acylaminocarbonsäuren durch Acylieren der entsprechenden Aminocarbonsäuren bzw. von Salzen dieser Säuren, dadurch gekennzeichnet, dass man
a) eine Aminocarbonsäure mit einem Salz einer Carbonsäure oder
b) ein Salz einer Aminocarbonsäure mit einer Carbonsäure oder
c) eine Aminocarbonsäure mit einer Carbonsäure in Gegenwart einer äquivalenten Menge eines Alkali- oder Erdalkalihydroxids oder -carbonats
bei Temperaturen von 100 bis 250 °C umsetzt, wobei die Aminocarbonsäure eine aliphatische Aminocarbonsäure mit wenigstens 3 C-Atomen im Molekül und die Carbonsäure eine aliphatische Carbonsäure mit 8 bis 22 C-Atomen ist.

Aufgabe der Erfindung war es, ein Verfahren zur Herstellung von Tensiden bereitzustellen, bei dem mindestens einer der Nachteile des Standes der Technik überwunden wird.

### Beschreibung der Erfindung

Überraschenderweise wurde gefunden, dass das im Folgenden beschriebene Verfahren die der Erfindung gestellte Aufgabe zu lösen vermag.

Gegenstand der vorliegenden Erfindung ist ein Verfahren umfassend die Verfahrensschritte
A) Bereitstellen einer Mischung umfassend eine Aminosäure und ein Amin ausgewählt aus mindestens einem aus der Gruppe bestehend aus Amidaminen und Trialkylaminen und
B) Umsetzen der Aminosäure mit einem Acylgruppendonor unter Erhalt einer N-Acylaminosäure,
   dadurch gekennzeichnet,
   dass das Trialkylamin ausgewählt ist aus solchen der allgemeinen Formel 2): 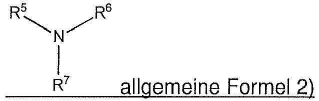
   mit R⁵ = gegebenenfalls hydroxysubstituierter, gegebenenfalls ein- oder mehrfach ungesättigter Alkyl- oder Alkylarylrest umfassend 6 bis 30, bevorzugt 8 bis 22, besonders bevorzugt 8 bis 18, Kohlenstoffatome,
   R⁶ und R⁷ = unabhängig voneinander gleich oder verschieden Alkylrest umfassend 1 bis 6 Kohlenstoffatome, bevorzugt Methyl.

Ein Vorteil der vorliegenden Erfindung ist, dass die nach dem erfindungsgemäßen Verfahren erhältlichen Tenside - wenn überhaupt - nur sehr geringe Mengen an freiem Glycerin enthalten. Größere Mengen Glycerin sind in kosmetischen Formulierungen unerwünscht weil diese beispielsweise die Verdickbarkeit von kosmetischen Formulierungen negativ beeinflussen.
Ein weiterer Vorteil der vorliegenden Erfindung ist, dass die nach dem erfindungsgemäßen Verfahren erhältlichen Tenside - wenn überhaupt - nur sehr geringe Mengen an NaCl enthalten. Dies ist von Vorteil, da höhere Mengen an NaCl korrosionsfördernd wirken können und da in einigen Formulierungen Elektrolyte unerwünscht sind.
Noch ein Vorteil der vorliegenden Erfindung ist, dass keine großen Mengen an basischem Metallkatalysator eingesetzt werden, die in dem Tensid verbleibt und später gegebenenfalls neutralisiert werden muss.
Ein weiterer Vorteil der vorliegenden Erfindung ist, dass in den nach dem erfindungsgemäßen Verfahren erhältlichen Tensiden nur sehr wenige oligopeptidische Nebenprodukte enthalten sind. Noch ein Vorteil der vorliegenden Erfindung ist, dass das erfindungsgemäße Verfahren bei niedrigeren Temperaturen durchgeführt werden kann und somit energetisch günstig durchzuführen ist.
Ein weiterer Vorteil ist, dass das erfindungsgemäße Verfahren gegenüber dem Standardverfahren per Schotten-Baumann-Route umweltschonender, weniger gefährlich und kostengünstiger ist

Ein weiterer Vorteil des erfindungsgemäßen Verfahrens ist, dass beispielsweise Cocoyl Glycinate aus verschiedensten Estern und Triglyceriden mit unterschiedlichen C-Kettenverteilungen hergestellt werden können, insbesondere auch ungesättigte Acylreste enthaltende, die über die Schotten-Baumann-Route nicht zugänglich sind. Dies kann sich auch positiv auf die Verdickungseigenschaften in kosmetischen Formulierungen auswirken.
Ein weiterer Vorteil ist, dass das Amin in Verfahrensschritt A) als Lösungsmittel basisch wirkt, wodurch die Reaktion zusätzlich katalysiert wird.
Ein weiterer Vorteil ist, dass das durch das erfindungsgemäße Verfahren hergestellte Produktgemisch aus Acylaminosäure und Amphotensid in deutlich höherer Konzentration in wässriger Lösung herstellbar ist als eine reine Acylaminosäure, wodurch u.a. Transportkosten eingespart werden können.

Ein weiterer Vorteil ist, dass die Acylaminosäure für das Amin in Verfahrensschritt A) als Lösungsvermittler in wässriger Lösung fungiert, wodurch eine anschließende Betainisierung effektiv durchgeführt werden kann.

Das erfindungsgemäße Verfahren ist insbesondere zur Herstellung von Tensiden, insbesondere von N-Acylaminosäuren, geeignet.
Unter dem Begriff "Säure" wird im Zusammenhang mit der vorliegenden Erfindung auch immer das korrespondierende Salz der Säure mitumfasst. Dies gilt insbesondere auch für die Begriffe "Aminosäure" und "N-Acylaminosäure".
Unter dem Begriff "Aminosäure" wird im Zusammenhang mit der vorliegenden Erfindung eine Verbindung aufweisend eine gegebenenfalls protonierte NH₂-Gruppe und eine gegebenenfalls deprotonierte Brønsted-Säuregruppe, insbesondere eine Carboxylat- oder Sulfonatgruppe, wobei eine Carboxylatgruppe besonders bevorzugt ist, verstanden.
Unter dem Begriff "Amidamin" wird im Zusammenhang mit der vorliegenden Erfindung ein gegebenenfalls protoniertes primäres, sekundäres oder tertiäres Amin aufweisend eine AmidGruppe, 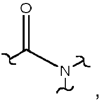 verstanden.
Alle angegebenen Prozent (%) sind, wenn nicht anders angegeben, Massenprozent.

Beansprucht wird ein Verfahren umfassend die Verfahrensschritte A) Bereitstellen einer Mischung umfassend eine Aminosäure und ein Amin ausgewählt aus mindestens einem aus der Gruppe bestehend aus Amidaminen und Trialkylaminen und B) Umsetzen der Aminosäure mit einem Acylgruppendonor unter Erhalt einer N-Acylaminosäure.

Es ist offenbar, dass auch jeweils mehrere der einzelnen Komponenten (Aminosäure, Amidamin, Trialkylamin und Acylgruppendonor), z.B. in Form von technischen Mischungen, eingesetzt werden können.

Die in der in Verfahrensschritt A) bereitgestellten Mischung enthaltene Aminosäure wird bevorzugt in Form einer wässrigen Lösung bereitgestellt.
Bevorzugt weist die in Verfahrensschritt A) bereitgestellten Mischung zusätzlich enthaltend Wasser einen pH-Wert in einem Bereich von 7 bis 14, bevorzugt 9 bis 13, auf. Die pH-Wert Einstellung erfolgt erfindungsgemäß bevorzugt durch die Zugabe von Ammoniak, basischen Erd- oder Alkalimetallsalzen, besonders bevorzugt Hydroxiden, ganz besonders bevorzugt KOH oder NaOH. In diesem Zusammenhang ist es insbesondere bevorzugt, dass die in Verfahrensschritt A) bereitgestellten Mischung zusätzlich enthaltend Wasser eine Erd- oder Alkalihydroxidkonzentration von 1,0 bis 4,0, bevorzugt von 1,01 bis 2,5, Moläquivalente bezogen auf die Aminosäure aufweist. Dies hat zur Folge, dass die in Verfahrensschritt A) bereitgestellte Aminosäure überwiegend in Form ihres Salzes vorliegt.
Die in Verfahrensschritt A) bereitgestellte Aminosäure ist erfindungsgemäß bevorzugt ausgewählt aus der Gruppe umfassend Peptide, Oligopeptide, proteinogene Aminosäuren, nicht-proteinogene Aminocarbonsäuren und Aminosulfonsäuren.
Erfindungsgemäß bevorzugt in Verfahrensschritt A) eingesetzte, nicht-proteinogene Aminocarbonsäuren sind ausgewählt aus L-Thyroxin, 2,6-Diaminopimelinsäure, L-Azetidin-2-carbonsäure, Sarkosin, Homoserin, Lanthionin, Djenkolsäure, Cystathionin, L-Homocystein, Ethionin, δ-Aminolävulinsäure, 4-Aminobenzoesäure, Dehydroalanin, GABA, 3-Aminoisobuttersäure, L-Homoserin, L-Ornithin, LCitrullin, Argininosuccinat, L-DOPA, L-5-Hydroxytryptophan, β-Alanin, β-Methylamino-Alanin, Ibotensäure, D-Valin, D-Alanin, D-Glutaminsäure, Hypoglycin, Hydroxyprolin, Norleucin, Methyltaurin und Taurin wobei Sarkosin, Methyltaurin und Taurin besonders bevorzugt sind.
Erfindungsgemäß bevorzugt in Verfahrensschritt A) eingesetzte, proteinogene Aminosäuren sind ausgewählt aus der Gruppe Glycin, Alanin, Valin und Glutaminsäure.
Erfindungsgemäß besonders bevorzugt in Verfahrensschritt A) eingesetzte Aminosäuren sind ausgewählt aus der Gruppe Sarkosin, Glycin und Glutaminsäure.

Als Amin wird in Verfahrensschritt A) bevorzugt mindestens ein Amidamin eingesetzt. Erfindungsgemäß bevorzugt in Verfahrensschritt A) eingesetztes Amidamin ist ausgewählt aus solchen der allgemeinen Formel 1): 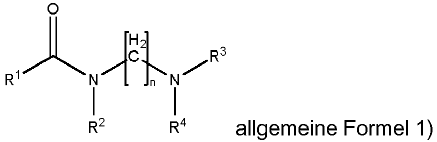
mit R¹ = gegebenenfalls hydroxysubstituierter, gegebenenfalls ein- oder mehrfach ungesättigter Alkyl- oder Alkylarylrest umfassend 5 bis 29, bevorzugt 7 bis 21, besonders bevorzugt 7 bis 17, Kohlenstoffatome,
mit R² = H oder Alkylrest umfassend 1-3, Kohlenstoffatome, bevorzugt H
mit R³ und R⁴ = unabhängig voneinander gleich oder verschieden H oder Alkylrest umfassend 1 bis 6 Kohlenstoffatome, bevorzugt Methyl und
n = 1 bis 6, bevorzugt 2 bis 3, besonders bevorzugt 3.

Besonders bevorzugt eingesetztes Amidamin der allgemeinen Formel 1) ist ausgewählt aus solchen mit R¹CO ausgewählt aus Acylresten der Fettsäuren, insbesondere Kokosfettsäuren, Palmkernfettsäuren, Palmfettsäuren, Rapsölfettsäuren. Solche von Fettsäuren abstammenden Amidamine sind bevorzugt dadurch gekennzeichnet, dass R² = H, R³ und R⁴ = Methyl und n=3.

Erfindungsgemäß bevorzugt in Verfahrensschritt A) eingesetztes Trialkylamin ist ausgewählt aus solchen der allgemeinen Formel 2): 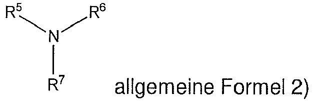
mit R⁵ = gegebenenfalls hydroxysubstituierter, gegebenenfalls ein- oder mehrfach ungesättigter Alkyl- oder Alkylarylrest umfassend 8 bis 22, besonders bevorzugt 8 bis 18, Kohlenstoffatome,
R⁶ und R⁷ = unabhängig voneinander gleich oder verschieden Alkylrest umfassend 1 bis 6 Kohlenstoffatome, bevorzugt Methyl.

Weist die in Verfahrensschritt A) bereitgestellten Mischung zusätzlich Wasser auf, so ist es erfindungsgemäß bevorzugt, dass das Wasser vor Durchführung von Verfahrensschritt B) auf einen Gehalt von unter 0,5 Gew.-%, insbesondere von unter 0,1 Gew.-%, reduziert wird, wobei sich die Gew.-% auf die Summe von Aminosäure, Amin und Wasser beziehen. Bevorzugt wird das Reduzieren des Wassergehaltes durch Erwärmen auf einen Temperaturbereich von 40 °C bis 130 °C, bevorzugt 80 bis 110 °C, durchgeführt, wobei es erfindungsgemäß insbesondere bevorzugt ist, in einem Druckbereich von 0,01 bis 1000 mbar, bevorzugt von 1 bis 400 mbar, zu arbeiten.

Erfindungsgemäß bevorzugt wird in Verfahrensschritt B) ein Acylgruppendonor ausgewählt aus Carbonsäureestern, bevorzugt Estern auf Basis von Alkanolen und Polyolen mit bis zu 6 C-Atomen, besonders bevorzugt mit bis zu 3 C-Atomen, ganz besonders bevorzugt Glycerinestern, eingesetzt.
Erfindungsgemäß bevorzugt wird in Verfahrensschritt B) ein Acylgruppendonor ausgewählt aus Acylgruppendonoren, die eine Acylgruppe ausgewählt ist aus mindestens einer aus der Gruppe der Acylgruppe von Fettsäuren bereitstellen, eingesetzt.
Insbesondere erfindungsgemäß bevorzugt wird in Verfahrensschritt B) ein Acylgruppendonor ausgewählt aus natürlichen Fetten und Ölen, bevorzugt ausgewählt aus Kokosfett, Palmkernöl und Palmöl, eingesetzt.
Erfindungsgemäß bevorzugt wird Verfahrensschritt B) in einem Temperaturbereich von 80 °C bis 160 °C, bevorzugt von 110 °C bis 150 °C, durchgeführt, wobei es erfindungsgemäß insbesondere bevorzugt ist, in einem Druckbereich von 1 bis 3000 mbar bevorzugt von 900 bis 1100 bar, zu arbeiten.
Erfindungsgemäß bevorzugt wird Verfahrensschritt B) in Gegenwart eines basischen Katalysators, bevorzugt Erd- oder Alkalimetallsalze, bevorzugt Hydroxide, insbesondere bevorzugt NaOH oder KOH, durchgeführt.

Es ist erfindungsgemäß bevorzugt, wenn das erfindungsgemäße Verfahren dadurch gekennzeichnet ist, dass die Komponenten Aminosäure, Amin und die Summe der Acylgruppen des Acylgruppendonors in einem Molverhältnis von 1 bis 100 zu 1 bis 100 zu 1 bis 100, bevorzugt von 1 bis 8 zu 1 bis 8 zu 1 bis 8, besonders bevorzugt von 1 bis 2 zu 1 bis 2 zu 1 bis 2, eingesetzt werden.

Das erfindungsgemäße Verfahren lässt sich bevorzugt um den Verfahrensschritt C) Betainisierung des Amins mit einer Halogencarbonsäure ergänzen.
Dieses bevorzugte erfindungsgemäße Verfahren umfassend Verfahrensschritt C) ist insbesondere zur Herstellung von Tensidmischungen umfassend mindestens eine N-Acylaminosäure und mindestens ein Betain geeignet.
Unter den in Verfahrensschritt C) eingesetzten Halogencarbonsäuren sind im allgemeinen Chlor- oder Bromcarbonsäuren mit 2 bis 4 Kohlenstoffatomen, vorzugsweise Chloressig- oder Chlorpropionsäure, insbesondere Chloressigsäure, zu verstehen. Neben den Halogencarbonsäuren können auch deren Alkalisalze, vorzugsweise deren Natriumsalze, eingesetzt werden.
Verfahrensschritt C) wird bevorzugt unter Einhaltung eines pH-Wertes im Bereich von 7 bis 13, bevorzugt von 7,5 bis 10, durchgeführt.
Verfahrensschritt C) wird bevorzugt in einem Temperaturbereich von 40 °C bis 120 °C, bevorzugt von 80 °C bis 115 °C, durchgeführt.
Verfahrensschritt C) wird bevorzugt bis zu einem Gehalt an freiem Amin von unter 0,5 Gew.-% durchgeführt, wobei sich die Gew.-% auf die Gesamtmenge in Verfahrensschritt A) eingesetztes Amin beziehen.
Es ist erfindungsgemäß bevorzugt, in Verfahrensschritt C) Wasser als Lösungsmittel zuzusetzen, insbesondere in einem Gewichtsverhältnis von Wasser zu Amin von 1 zu 0,01 bis 0,01 zu 1, bevorzugt von 1 zu 0,05 bis 1 zu 1, besonders bevorzugt von 1 zu 0,1 bis 1 zu 0,6.

### Beispiele:

### Beispiel 1:

In einer 1 L Rührapparatur mit Destillationsaufsatz und Stickstoffeinleitung wurden zu einer Lösung aus 37,5 g Glycin (0,5 mol), 22,4 g NaOH (0,56 mol) und 67,2 g Wasser 133,9 g eines Dimethylaminopropylamides von Kokosfettsäure gegeben (0,46 mol, hergestellt nach der Methoden wie in EP656346 beschrieben). Die Mischung wurde auf 105 °C unter Vakuum erhitzt, bis dem System alles Wasser entzogen war. Anschließend wurden 112,8 g Kokosfett (0,17 mol) zugegeben und die Mischung auf 145 °C geheizt. Nach 4 h Reaktionszeit war der Umsatz nach DC Kontrolle (n-Butanol:Essigsäure:Wasser = 3:1:1, Ninhydrin-Färbereagenz, R_{f}=0,2) vollständig. Die Mischung wurde durch NMR-Spektroskopie charakterisiert und enthielt zu diesem Zeitpunkt 45 % Cocoylglycinat (bestätigt durch HPLC: Standard Lauroyl glycinat).
Es wurden 112,7 g des Reaktionsguts (0,18 mol Cocoylglycinat, 0,17 mol Amidamin) in 268,6 g Wasser gelöst und mit 19,9 g Monochloressigsäure (80 %ige wässrige Lösung, 0,17 mol) bei 50-95 °C gemäß Standardverfahren umgesetzt und der pH-Wert auf 9,25 eingestellt. Nach 4 h bei 95 °C war der Gehalt an restlichem Amidamin unter 0,2 %. Das Produkt wurde über NMR-Spektroskopie charakterisiert, die wässrige Lösung enthielt zu diesem Zeitpunkt rechnerisch 13 % Cocoylglycinat (bestätigt durch HPLC: Standard Lauroylglycinat).

### Beispiel 2:

Gemäß Beispiel 1 wurden zu einer Lösung aus 40,5 g Glycin (0,54 mol), 48,6 g NaOH (0,59 mol) und 72,9 g Wasser 49,5 g eines Dimethylaminopropylamides von Kokosfettsäure gegeben (0,17 mol, hergestellt nach den bekannten Methoden wie beispielsweise in EP656346). Die Mischung wurde auf 105 °C unter Vakuum erhitzt, bis dem System alles Wasser entzogen war. Es wurden 13,5 g NaOMe (25% in Methanol, 0,05 mol NaOMe) zugegeben und Methanol verdampft. Anschließend wurden 121,8 g Kokosfett (0,18 mol). zugegeben und die Mischung auf 145 °C geheizt. Nach 4 h Reaktionszeit war der Umsatz nach DC Kontrolle (n-Butanol:Essigsäure:Wasser = 3:1:1, Ninhydrin-Färbereagenz, R_{f}=0,2) vollständig.
Es wurden 88,5 g des Reaktionsguts (0,19 mol Cocoylglycinat, 0,07 mol Amidamin) in 88,5 g Wasser gelöst und mit einer Lösung aus 8,2 g Monochloressigsäure (80 %ige wässrige Lösung, 0,07 mol) in 119,5 g Wasser bei 50-95 °C gemäß Standardverfahren umgesetzt und der pH-Wert auf 9,25 eingestellt. Nach 4 h bei 95 °Cwar der Gehalt an restlichem Amidamin unter 0,2 %. Das Produkt wurde über NMR-Spektroskopie charakterisiert, die wässrige Lösung enthielt zu diesem Zeitpunkt rechnerisch 19 % Cocoylglycinat (bestätigt durch HPLC: Standard Lauroylglycinat).

### Beispiel 3:

Gemäß Beispiel 1 wurden zu einer Lösung aus 18,8 g Glycin (0,25 mol), 20 g NaOH (0,25 mol) und 38,1 g Wasser 178,1 g eines Dimethylaminopropylamides von Kokosfettsäure gegeben (0,61 mol, hergestellt nach den bekannten Methoden wie beispielsweise in EP656346). Die Mischung wurde auf 105 °C unter Vakuum erhitzt, bis dem System alles Wasser entzogen war. Anschließend wurden 53,6 g Laurinsäuremethylester (0,25 mol). zugegeben und die Mischung auf 130°C geheizt. Nach 4 h Reaktionszeit war der Umsatz nach DC Kontrolle (n-Butanol:Essigsäure:Wasser = 3:1:1, Ninhydrin-Färbereagenz, R_{f}=0,2) vollständig.

Es wurden 78,9 g des Reaktionsguts (0,07 mol Lauroylglycinat, 0,16 mol Amidamin) in 221,2 g Wasser gelöst und mit 18,8 g Monochloressigsäure (80 %ige wässrige Lösung, 0,16 mol) bei 50-95 °C gemäß Standardverfahren umgesetzt und der pH-Wert auf 9,25 eingestellt. Nach 4 h war der Gehalt an restlichem Amidamin unter 0,2 %. Das Produkt wurde über NMR-Spektroskopie charakterisiert, die wässrige Lösung enthielt zu diesem Zeitpunkt rechnerisch 6 % Lauroylglycinat (HPLC: Standard Lauroyl glycinat).

### Beispiel 4:

Gemäß Beispiel 1 wurden zu einer Lösung aus 44,5 g Sarkosin (0,5 mol), 22,4 g NaOH (0,56 mol) und 67,2 g Wasser 133,9 g eines Dimethylaminopropylamides von Kokosfettsäure gegeben (0,46 mol, hergestellt nach den bekannten Methoden wie beispielsweise in EP656346). Die Mischung wurde auf 105 °C unter Vakuum erhitzt, bis dem System alles Wasser entzogen war. Anschließend wurden 112,8 g Kokosfett (0,17 mol) zugegeben und die Mischung auf 145 °C geheizt. Nach 4 h Reaktionszeit war der Umsatz nach DC Kontrolle (n-Butanol:Essigsäure:Wasser = 3:1:1, Ninhydrin-Färbereagenz, R_{f}=0,2) vollständig.
Es wurden 112,7 g des Reaktionsguts (0,18 mol Cocoylsarkosin, 0,16 mol Amidamin) in 268,6 g Wasser gelöst und mit 18,9 g Monochloressigsäure (80 %ige wässrige Lösung, 0,16 mol) bei 50-95 °C gemäß Standardverfahren umgesetzt und der pH-Wert auf 9,25 eingestellt. Nach 4 h war der Gehalt an restlichem Amidamin unter 0,2 %. Das Produkt wurde über NMR-Spektroskopie charakterisiert, die wässrige Lösung enthielt zu diesem Zeitpunkt rechnerisch 14 % Cocoylsarkosinat.

### Beispiel 5:

Gemäß Beispiel 1 wurden zu einer Lösung aus 60,1 g Glycin (0,8 mol), 35,2 g NaOH (0,88 mol) und 105,6 g Wasser 133,9 g eines Dimethylaminopropylamides von Kokosfettsäure gegeben (0,46 mol, hergestellt nach den bekannten Methoden wie beispielsweise in EP656346). Die Mischung wurde auf 105 °C unter Vakuum erhitzt, bis dem System alles Wasser entzogen war. Anschließend wurden 136,2 g Triglyceride der Capryl-/Caprinsäure (TEGOSOFT® CT, 0,27 mol) zugegeben und die Mischung auf 145 °C geheizt. Nach 4 h Reaktionszeit war der Umsatz nach DC Kontrolle (n-Butanol:Essigsäure:Wasser = 3:1:1, Ninhydrin-Färbereagenz, R_{f}=0,2) vollständig.
Es wurden 112,7 g des Reaktionsguts (0,26 mol Capryloyl-/Caprinoylglycinat, 0,15 mol Amidamin) in 268,6 g Wasser gelöst und mit 17,7 g Monochloressigsäure (80 %ige wässrige Lösung, 0,15 mol) bei 50-95 °C gemäß Standardverfahren umgesetzt und der pH-Wert auf 9,25 eingestellt. Nach 4 h war der Gehalt an restlichem Amidamin unter 0,2 %. Das Produkt wurde über NMR-Spektroskopie charakterisiert, die wässrige Lösung enthielt zu diesem Zeitpunkt rechnerisch 14 % Capryloyl-/Caprinoylglycinat.

### Beispiel 6:

Gemäß Beispiel 1 wurden zu einer Lösung aus 11,3 g Glycin (0,15 mol), 6,8 g NaOH (0,17 mol) und 20,4 g Wasser 64 g *N*,*N*-Dimethyldodecylamin gegeben (0,3 mol). Die Mischung wurde auf 105 °C unter Vakuum erhitzt, bis dem System alles Wasser entzogen war. Anschließend wurden 33,8 g Kokosfett (0,05 mol) zugegeben und die Mischung auf 145 °C geheizt. Nach 4 h Reaktionszeit war der Umsatz nach DC Kontrolle (n-Butanol:Essigsäure:Wasser = 3:1:1, Ninhydrin-Färbereagenz, R_{f}=0,2) vollständig.
Es wurden 70 g des Reaktionsguts (0,19 mol Cocoylglycinat, 0,17 mol *N*,*N*-Dimethyldodecylamin) in 250 g Wasser gelöst und mit 20,1 g Monochloressigsäure (80 %ige wässrige Lösung, 0,17 mol) bei 50-95 °C umgesetzt und der pH-Wert auf 9,25 eingestellt. Das Produkt wurde über NMR-Spektroskopie charakterisiert, die wässrige Lösung enthielt zu diesem Zeitpunkt rechnerisch 15 % Cocoylglycinat.

### Beispiel 7:

Gemäß Beispiel 1 wurden zu einer Lösung aus 37,5 g Glycin (0,5 mol), 31,4 g KOH (0,56 mol) und 94,1 g Wasser 137,1 g eines Dimethylaminopropylamides von Palmkernfettsäure gegeben (0,46 mol, hergestellt nach den bekannten Methoden wie beispielsweise in EP656346). Die Mischung wurde auf 105 °C unter Vakuum erhitzt, bis dem System alles Wasser entzogen war. Anschließend wurden 113 g Palmkernfett (0,17 mol) zugegeben und die Mischung auf 135 °C geheizt. Nach 4 h Reaktionszeit war der Umsatz nach DC Kontrolle (n-Butanol:Essigsäure:Wasser = 3:1:1, Ninhydrin-Färbereagenz, R_{f}=0,2) vollständig.
Es wurden 117,1 g des Reaktionsguts (0,18 mol Cocoylglycinat, 0,17 mol Amidamin) in 268,6 g Wasser gelöst und mit 19,9 g Monochloressigsäure (80 %ige wässrige Lösung, 0,17 mol) bei 50-95 °C gemäß Standardverfahren umgesetzt und der pH-Wert auf 9,25 eingestellt. Nach 4 h war der Gehalt an restlichem Amidamin unter 0,2 %. Das Produkt wurde über NMR-Spektroskopie charakterisiert, die wässrige Lösung enthielt zu diesem Zeitpunkt rechnerisch 17 % Cocoylglycinat.

### Beispiel 8:

Gemäß Beispiel 1 wurden zu einer Lösung aus 24,4 g Glycin (0,33 mol), 14,7 g NaOH (0,37 mol) und 44,3 g Wasser 93,2 g *Tripropylamin* gegeben (0,65 mol). Die Mischung wurde auf 110 °C unter Vakuum erhitzt, bis dem System alles Wasser entzogen war. Anschließend wurden 73,3 g Kokosfett (0,11 mol) zugegeben und die Mischung auf 145 °C geheizt. Nach 4 h Reaktionszeit war der Umsatz nach DC Kontrolle (n-Butanol:Essigsäure:Wasser = 3:1:1, Ninhydrin-Färbereagenz, R_{f}=0,2) nicht vollständig. Die Produktmischung enthielt zu diesem Zeitpunkt 1,4 % Cocoylglycinat (HPLC: Standard Lauroylglycinat). Der theoretische Wert für den Anteil an Cocoylglycinat zu diesem Zeitpunkt beträgt 46,6 %.

### Beispiel 9:

Gemäß Beispiel 1 wurden zu einer Lösung aus 21,4 g Glycin (0,29 mol), 12,9 g NaOH (0,32 mol) und 38,8 g Wasser 121,7 g *N*,*N*-Dimethyldodecylamin gegeben (0,57 mol). Die Mischung wurde auf 105 °C unter Vakuum erhitzt, bis dem System alles Wasser entzogen war. Anschließend wurden 55,3 g Kokosfettsäure (0,29 mol) zugegeben und die Mischung auf 145 °C geheizt. Nach 4 h Reaktionszeit war der Umsatz nach DC Kontrolle (n-Butanol:Essigsäure:Wasser = 3:1:1, Ninhydrin-Färbereagenz, R_{f}=0,2) nicht vollständig. Die Produktmischung enthielt zu diesem Zeitpunkt 1,6 % Cocoylglycinat (HPLC: Standard Lauroylglycinat). Der theoretische Wert für den Anteil an Cocoylglycinat zu diesem Zeitpunkt beträgt 17,9 %.

## Patentansprüche

1. Verfahren umfassend die Verfahrensschritte
A) Bereitstellen einer Mischung umfassend
eine Aminosäure und
ein Amin ausgewählt aus mindestens einem aus der Gruppe bestehend aus Amidaminen und Trialkylaminen und
B) Umsetzen der Aminosäure mit einem Acylgruppendonor unter Erhalt einer N-Acylaminosäure,
**dadurch gekennzeichnet, dass** das Trialkylamin ausgewählt ist aus solchen der allgemeinen Formel 2): 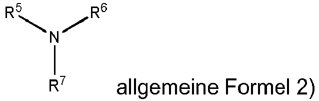
mit R⁵ = gegebenenfalls hydroxysubstituierter, gegebenenfalls ein- oder mehrfach ungesättigter Alkyl- oder Alkylarylrest umfassend 6 bis 30, bevorzugt 8 bis 22, besonders bevorzugt 8 bis 18, Kohlenstoffatome,
R⁶ und R⁷ = unabhängig voneinander gleich oder verschieden Alkylrest umfassend 1 bis 6 Kohlenstoffatome, bevorzugt Methyl.

2. Verfahren nach Anspruch 1, **dadurch gekennzeichnet, dass** die Aminosäure ausgewählt ist aus der Gruppe umfassend Peptide, Oligopeptide, proteinogene Aminosäuren, nicht-proteinogene Aminocarbonsäuren und Aminosulfonsäuren.

3. Verfahren nach Anspruch 1 oder 2, **dadurch gekennzeichnet, dass** die Aminosäure ausgewählt ist aus der Gruppe Sarkosin, Methyltaurin, Taurin, Glycin, Alanin, Valin und Glutaminsäure.

4. Verfahren nach mindestens einem der vorgenannten Ansprüche, **dadurch gekennzeichnet, dass** das Amin ein Amidamin ist, ausgewählt aus solchen der allgemeinen Formel 1) 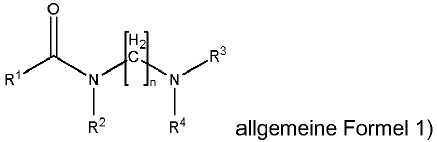
mit R¹ = gegebenenfalls hydroxysubstituierter, gegebenenfalls ein- oder mehrfach ungesättigter Alkyl- oder Alkylarylrest umfassend 5 bis 29, bevorzugt 7 bis 21, besonders bevorzugt 7 bis 17, Kohlenstoffatome,
mit R² = H oder Alkylrest umfassend 1-3, Kohlenstoffatome, bevorzugt H
mit R³ und R⁴ = unabhängig voneinander gleich oder verschieden H oder Alkylrest umfassend 1 bis 6 Kohlenstoffatome, bevorzugt Methyl und
n = 1 bis 6, bevorzugt 2 bis 3, besonders bevorzugt 3.

5. Verfahren nach mindestens einem der Ansprüche 1 bis 3, **dadurch gekennzeichnet, dass** das Amin ein Trialkylamin ist ausgewählt aus solchen der allgemeinen Formel 2): 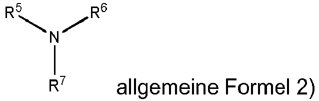
mit R⁵ = gegebenenfalls hydroxysubstituierter, gegebenenfalls ein- oder mehrfach ungesättigter Alkyl- oder Alkylarylrest umfassend 8 bis 22, besonders bevorzugt 8 bis 18, Kohlenstoffatome,
R⁶ und R⁷ = unabhängig voneinander gleich oder verschieden Alkylrest umfassend 1 bis 6 Kohlenstoffatome, bevorzugt Methyl.

6. Verfahren nach mindestens einem der vorgenannten Ansprüche, **dadurch gekennzeichnet, dass** die in Verfahrensschritt A) bereitgestellten Mischung zusätzlich Wasser aufweist und das Wasser vor Durchführung von Verfahrensschritt B) auf einen Gehalt von unter 0,5 Gew.-%, insbesondere von unter 0,1 Gew.-%, reduziert wird, wobei sich die Gew.-% auf die Summe von Aminosäure, Amin und Wasser beziehen.

7. Verfahren nach mindestens einem der vorgenannten Ansprüche, **dadurch gekennzeichnet, dass** in Verfahrensschritt B) ein Acylgruppendonor ausgewählt aus Acylgruppendonoren, die eine Acylgruppe ausgewählt aus mindestens einer aus der Gruppe der Acylgruppe von Fettsäuren bereitstellen, ausgewählt aus natürlichen Fetten und Ölen, eingesetzt wird.

8. Verfahren nach mindestens einem der vorgenannten Ansprüche, **dadurch gekennzeichnet, dass** Verfahrensschritt B) in einem Temperaturbereich von 80 °C bis 160 °C, bevorzugt von 110 °C bis 150 °C, durchgeführt wird, insbesondere in einem Druckbereich von 1 bis 3000 mbar bevorzugt von 900 bis 1100 bar.

9. Verfahren nach mindestens einem der vorgenannten Ansprüche, **dadurch gekennzeichnet, dass** die Komponenten Aminosäure, Amin und die Summe der Acylgruppen des Acylgruppendonors in einem Molverhältnis von 1 bis 100 zu 1 bis 100 zu 1 bis 100, bevorzugt von 1 bis 8 zu 1 bis 8 zu 1 bis 8, besonders bevorzugt von 1 bis 2 zu 1 bis 2 zu 1 bis 2, eingesetzt werden.

10. Verfahren nach mindestens einem der vorgenannten Ansprüche weiter umfassend den Verfahrensschritt
C) Betainisierung des Amins mit einer Halogencarbonsäure.

11. Verfahren nach Anspruch 10, **dadurch gekennzeichnet, dass** die Halogencarbonsäure ausgewählt ist aus Chloressig- oder Chlorpropionsäure.

12. Verfahren nach Anspruch 10 oder 11, **dadurch gekennzeichnet, dass** Verfahrensschritt C) unter Einhaltung eines pH-Wertes im Bereich von 7 bis 13, bevorzugt von 7,5 bis 10, durchgeführt wird.

13. Verfahren nach mindestens einem der Ansprüche 10 bis 12, **dadurch gekennzeichnet, dass** Verfahrensschritt C) in einem Temperaturbereich von 40 °C bis 120 °C, bevorzugt von 80 °C bis 115 °C, durchgeführt wird.

14. Verfahren nach mindestens einem der Ansprüche 10 bis 13, **dadurch gekennzeichnet, dass** Verfahrensschritt C) bis zu einem Gehalt an freiem Amin von unter 0,5 Gew.-%, wobei sich die Gew.-% auf die Gesamtmenge in Verfahrensschritt A) eingesetztes Amin beziehen, durchgeführt wird.

15. Verfahren nach mindestens einem der Ansprüche 10 bis 14, **dadurch gekennzeichnet, dass** in Verfahrensschritt C) Wasser als Lösungsmittel, insbesondere in einem Gewichtsverhältnis von Wasser zu Amin von 1 zu 0,01 bis 0,01 zu 1, bevorzugt von 1 zu 0,05 bis 1 zu 1, besonders bevorzugt von 1 zu 0,1 bis 1 zu 0,6, zugesetzt wird.

## Claims

1. Method comprising the method steps of
A) providing a mixture comprising
an amino acid and
an amine selected from at least one of the group consisting of amidamines and trialkylamines and
B) reacting the amino acid with an acyl group donor to obtain an N-acylamino acid,
**characterized in that** the trialkylamine is selected from those of the general formula 2): 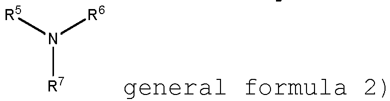
where R⁵ = optionally hydroxy-substituted, optionally mono- or polyunsaturated alkyl or alkylaryl radicals comprising 6 to 30, preferably 8 to 22, particularly preferably 8 to 18, carbon atoms,
R⁶ and R⁷ = mutually independent, identical or different alkyl radicals comprising 1 to 6 carbon atoms, preferably methyl.

2. Method according to Claim 1, **characterized in that** the amino acid is selected from the group comprising peptides, oligopeptides, proteinogenic amino acids, non-proteinogenic aminocarboxylic acids and aminosulphonic acids.

3. Method according to Claim 1 or 2, **characterized in that** the amino acid is selected from the group of sarcosine, methyltaurine, taurine, glycine, alanine, valine and glutamic acid.

4. Method according to at least one of the preceding claims, **characterized in that** the amine is an amidamine, selected from those of the general formula 1) 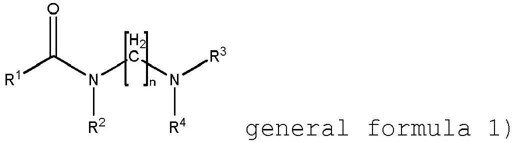
where R¹ = optionally hydroxy-substituted, optionally mono- or polyunsaturated alkyl or alkylaryl radicals comprising 5 to 29, preferably 7 to 21, particularly preferably 7 to 17, carbon atoms,
where R² = H or alkyl radical comprising 1-3 carbon atoms, preferably H
where R³ and R⁴ = mutually independent, identical or different H or alkyl radicals comprising 1 to 6 carbon atoms, preferably methyl and
n = 1 to 6, preferably 2 to 3, particularly preferably 3.

5. Method according to at least one of Claims 1 to 3, **characterized in that** the amine is a trialkylamine selected from those of the general formula 2): 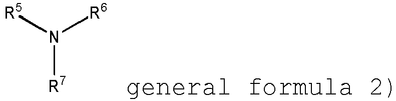
where R⁵ = optionally hydroxy-substituted, optionally mono- or polyunsaturated alkyl or alkylaryl radicals comprising 8 to 22, particularly preferably 8 to 18, carbon atoms,
R⁶ and R⁷ = mutually independent, identical or different alkyl radicals comprising 1 to 6 carbon atoms, preferably methyl.

6. Method according to at least one of the preceding claims, **characterized in that** the mixture provided in method step A) additionally comprises water and the water before carrying out method step B) is reduced to a content of below 0.5% by weight, in particular below 0.1% by weight, wherein the % by weight refers to the sum total of amino acid, amine and water.

7. Method according to at least one of the preceding claims, **characterized in that** in method step B) an acyl group donor is used selected from acyl group donors which provide an acyl group selected from at least one of the group of acyl groups of fatty acids selected from natural fats and oils.

8. Method according to at least one of the preceding claims, **characterized in that** method step B) is carried out in a temperature range from 80°C to 160°C, preferably from 110°C to 150°C, particularly in a pressure range from 1 to 3000 mbar, preferably from 900 to 1100 mbar.

9. Method according to at least one of the preceding claims, **characterized in that** the components amino acid, amine and the sum total of the acyl groups of the acyl group donor are used in a molar ratio of from 1:100:1 to 100:1:100, preferably from 1:8:1 to 8:1:8, particularly preferably from 1:2:1 to 2:1:2.

10. Method according to at least one of the preceding claims, further comprising the method step of C) betainization of the amine with a halocarboxylic acid.

11. Method according to Claim 10, **characterized in that** the halocarboxylic acid is selected from chloroacetic acid or chloropropionic acid.

12. Method according to Claim 10 or 11, **characterized in that** method step C) is carried out maintaining a pH in the range of 7 to 13, preferably 7.5 to 10.

13. Method according to at least one of Claims 10 to 12, **characterized in that** method step C) is carried out in a temperature range from 40 °C to 120 °C, preferably 80°C to 115°C.

14. Method according to at least one of Claims 10 to 13, **characterized in that** method step C) is carried out up to a free amine content of below 0.5% by weight, wherein the % by weight refers to the total amount of amine used in method step A).

15. Method according to at least one of Claims 10 to 14, **characterized in that** water is added as solvent in method step C), in particular in a weight ratio of water to amine of from 1:0.01 to 0.01:1, preferably from 1:0.05 to 1:1, particularly preferably from 1:0.1 to 1:0.6.

## Revendications

1. Procédé comprenant les étapes de procédé consistant à :
A) fournir un mélange comprenant
un acide aminé et
une amine choisie parmi au moins une du groupe constitué par les amidoamines et les trialkylamines et
B) faire réagir l'acide aminé avec un donneur de groupes acyle, avec obtention d'un acide N-acyl-aminé,
**caractérisé en ce que** la trialkylamine est choisie parmi celles de formule générale 2) : 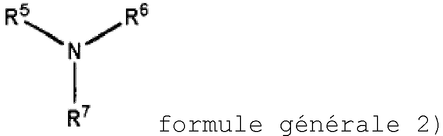
où R⁵ = un radical alkyle ou alkylaryle éventuellement une ou plusieurs fois insaturé, éventuellement substitué par hydroxy, comprenant 6 à 30, de préférence 8 à 22, de façon particulièrement préférée 8 à 18 atomes de carbone,
R⁶ et R⁷ = identiques ou différents, indépendamment l'un de l'autre, un radical alkyle comprenant 1 à 6 atomes de carbone, de préférence méthyle.

2. Procédé selon la revendication 1, **caractérisé en ce que** l'acide aminé est choisi dans le groupe comprenant les peptides, oligopeptides, acides aminés protéinogènes, acides aminocarboxyliques non protéinogènes et acides aminosulfoniques.

3. Procédé selon la revendication 1 ou 2, **caractérisé en ce que** l'acide aminé est choisi dans le groupe constitué par la sarcosine, la méthyltaurine, la taurine, la glycine, l'alanine, la valine et l'acide glutamique.

4. Procédé selon au moins l'une quelconque des revendications susmentionnées, **caractérisé en ce que** l'amine est une amidoamine choisie parmi celles de formule générale 1) 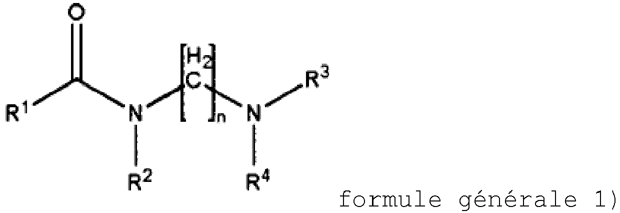
où R¹ = un radical alkyle ou alkylaryle éventuellement une ou plusieurs fois insaturé, éventuellement substitué par hydroxy, comprenant 5 à 29, de préférence 7 à 21, de façon particulièrement préférée 7 à 17 atomes de carbone,
où R² = H ou un radical alkyle comprenant 1-3 atomes d'hydrogène, de préférence H
où R³ et R⁴ = identiques ou différents, indépendamment l'un de l'autre, H ou un radical alkyle comprenant 1 à 6 atomes de carbone, de préférence méthyle et
n = 1 à 6, de préférence 2 à 3, de façon particulièrement préférée 3.

5. Procédé selon au moins l'une quelconque des revendications 1 à 3, **caractérisé en ce que** l'amine est une trialkylamine choisie parmi celles de formule générale 2) : 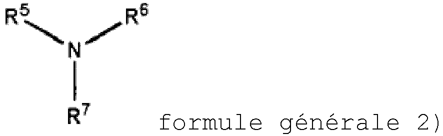
où R⁵ = un radical alkyle ou alkylaryle éventuellement une ou plusieurs fois insaturé, éventuellement substitué par hydroxy, comprenant 8 à 22, de façon particulièrement préférée 8 à 18 atomes de carbone,
R⁶ et R⁷ = identiques ou différents, indépendamment l'un de l'autre, un radical alkyle comprenant 1 à 6 atomes de carbone, de préférence méthyle.

6. Procédé selon au moins l'une quelconque des revendications susmentionnées, **caractérisé en ce que** le mélange fourni dans l'étape A) du procédé comporte en plus de l'eau et l'eau est réduite, avant l'excéution de l'étape B) du procédé, à une teneur de moins de 0,5 % en poids, en particulier de moins de 0,1 % en poids, les % en poids se rapportant à la somme d'acide aminé, amine et eau.

7. Procédé selon au moins l'une quelconque des revendications susmentionnées, **caractérisé en ce que** dans l'étape B) du procédé on utilise un donneur de groupes acyle choisi parmi les donneurs de groupes acyle qui fournissent un groupe acyle choisi parmi au moins un pris dans le groupe du groupe acyle d'acides gras choisis parmi les huiles et graisses naturelles.

8. Procédé selon au moins l'une quelconque des revendications susmentionnées, **caractérisé en ce qu'**on effectue l'étape B) du procédé dans une plage de température de 80 °C à 160 °C, de préférence de 110 °C à 150 °C, en particulier dans une plage de pression de 1 à 3 000 mbars, de préférence de 900 à 1 100 bars.

9. Procédé selon au moins l'une quelconque des revendications susmentionnées, **caractérisé en ce qu'**on utilise les composants acide aminé, amine et la somme des groupes acyle du donneur de groupes acyle en un rapport molaire de 1 à 100 : 1 à 100 : 1 à 100, de préférence de 1 à 8 : 1 à 8 : 1 à 8, de façon particulièrement préférée de 1 à 2 : 1 à 2 : 1 à 2.

10. Procédé selon au moins l'une quelconque des revendications susmentionnées, comprenant en outre l'étape de procédé
C) bétaïnisation de l'amine avec un acide halogénocarboxylique.

11. Procédé selon la revendication 10, **caractérisé en ce que** l'acide halogénocarboxylique est choisi parmi l'acide chloracétique et l'acide chloropropionique.

12. Procédé selon la revendication 10 ou 11, **caractérisé en ce qu'**on effectue l'étape C) du procédé en maintenant un pH dans l'intervalle de 7 à 13, de préférence de 7,5 à 10.

13. Procédé selon au moins l'une quelconque des revendications 10 à 12, **caractérisé en ce qu'**on effectue l'étape C) du procédé dans une plage de température de 40 °C à 120 °C, de préférence de 80 °C à 115 °C.

14. Procédé selon au moins l'une quelconque des revendications 10 à 13, **caractérisé en ce qu'**on effectue l'étape C) du procédé jusqu'à une teneur en amine libre de moins de 0,5 % en poids, les % en poids se rapportant à la quantité totale d'amine utilisée dans l'étape A) du procédé.

15. Procédé selon au moins l'une quelconque des revendications 10 à 14, **caractérisé en ce que** dans l'étape C) du procédé on ajoute de l'eau en tant que solvant, en particulier en un rapport pondéral d'eau à amine de 1 : 0,01 à 0,01 : 1, de préférence de 1 : 0,05 à 1 : 1, de façon particulièrement préférée de 1 : 0,1 à 1 : 0,6.
